(19) Europäisches Patentamt
European Patent Office
Office européen des brevets

(11) Veröffentlichungsnummer : **0 155 609**
**B1**

(12) # EUROPÄISCHE PATENTSCHRIFT

(45) Veröffentlichungstag der Patentschrift : **04.03.87**

(51) Int. Cl.[4] : **C 07 C143/86**

(21) Anmeldenummer : **85102697.1**

(22) Anmeldetag : **09.03.85**

(54) **Verfahren zur Herstellung kristalliner Salze des Acetoacetamid-N-sulfofluorids.**

(30) Priorität : **21.03.84 DE 3410233**

(43) Veröffentlichungstag der Anmeldung : **25.09.85 Patentblatt 85/39**

(45) Bekanntmachung des Hinweises auf die Patenterteilung : **04.03.87 Patentblatt 87/10**

(84) Benannte Vertragsstaaten : **AT BE CH DE FR GB IT LI NL SE**

(56) Entgegenhaltungen : **DE-A- 2 453 063**

(73) Patentinhaber : **HOECHST AKTIENGESELLSCHAFT**
**Postfach 80 03 20**
**D-6230 Frankfurt am Main 80 (DE)**

(72) Erfinder : **Linkies, Adolf, Dr.**
**Loreleistrasse 12**
**D-6230 Frankfurt am Main 80 (DE)**
Erfinder : **Reuschling, Dieter, Dr.**
**Beethovenstrasse 27**
**D-6308 Butzbach (DE)**

**EP 0 155 609 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Acetoacetamid-N-sulfofluorid ist die Verbindung der Formel

$$CH_3—CO—CH_2—CONHSO_2F.$$

Infolge des aciden Wasserstoffs am Stickstoffatom ist die Verbindung zur Salzbildung (mit Basen) befähigt. Die Salze — wie z. B. das Triethylammoniumsalz — sind hauptsächlich Zwischenprodukte, insbesondere zur Herstellung der nicht-toxischen Salze des 6-Methyl-3,4-dihydro-1,2,3-oxathiazin-4-on-2,2-dioxids, welche wegen ihres z. T. intensiven Süßgeschmacks als Süßstoffe auf dem Nahrungsmittelsektor verwendet werden können. Unter den nicht-toxischen Salzen des 6-Methyl-3,4-dihydro-1,2,3-oxathiazin-4-on-2,2-dioxids ist das Kaliumsalz (« Acesulfam K » oder auch nur « Acesulfam ») von besonderer Bedeutung. Etwa ausgehend von dem Triethylammoniumsalz des Acetoacetamid-N-sulfofluorids kommt man zu dem Süßstoff Acesulfam durch Umsetzung mit 2 Äquivalenten einer Kaliumbase wie z. B. KOH, vorzugsweise in methanolischer oder wäßriger Lösung :

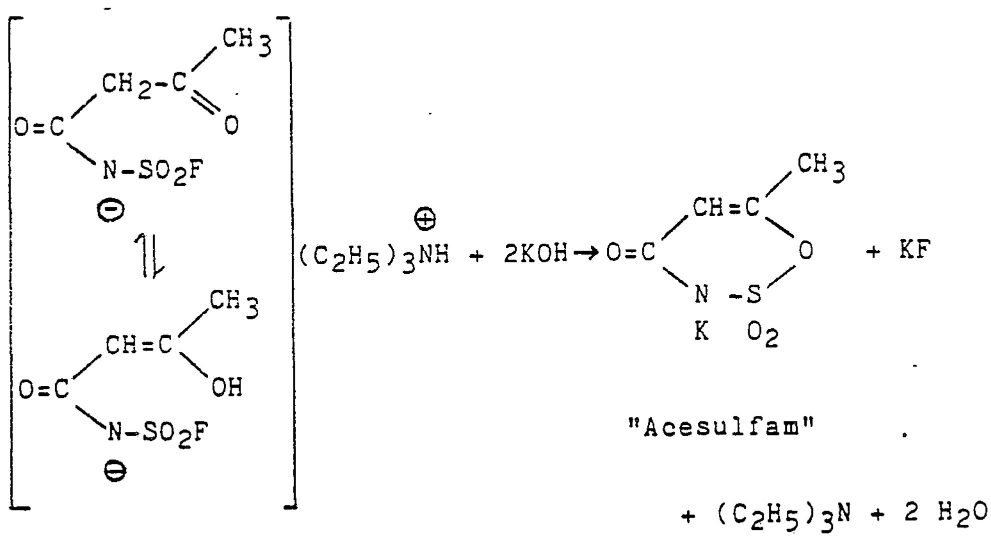


Es ist bekannt, die Salze des Acetoacetamid-N-sulfofluorids mit organischen N-Basen durch Umsetzung von Amidosulfofluorid $H_2NSO_2F$ mit den mindestens etwa äquimolaren Menge Diketen in Gegenwart von mindestens einem Grammäquivalent einer organischen Stickstoffbase pro Mol Amidosulfofluorid ggf. in einem inerten organischen Lösungsmittel bei Temperaturen zwischen etwa — 30 und + 100 °C, vorzugsweise etwa — 20 und + 50 °C, insbesondere zwischen etwa — 10 und + 30 °C, umzusetzen (DE-OS 2 453 063). Beispielsweise bei Verwendung von Triethylamin als organischer N-Base verläuft die Umsetzung nach folgendem Reaktionsschema :

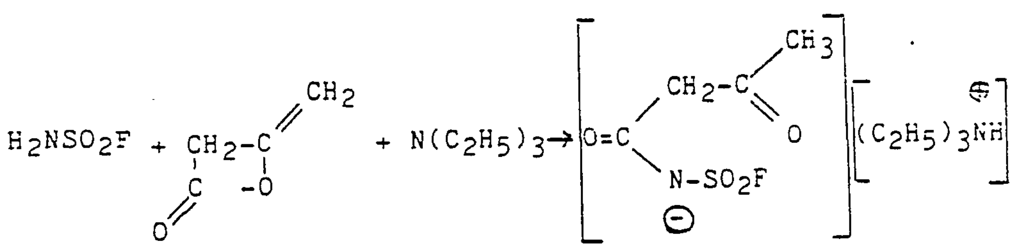

Die nach diesem Verfahren erhältlichen Salze des Acetoacetamid-N-sulfofluorids mit organischen N-Basen fallen meist als nicht-kristalline bräunliche Verbindungen von öliger Konsistenz — und nur in Ausnahmefällen in fester, kristalliner Form — an. Von den 12 Ausführungsbeispielen der DE-OS 2 453 063 wird nur in einem einzigen Fall (Beispiel 12) vom Erhalt eines festen, kristallinen Produkts (Tetramethylethylendiaminsalz des Acetoacetamid-N-sulfofluorids) berichtet.

Da die Entstehung nicht-kristalliner öliger Produkte praktisch immer einen gewissen Nachteil darstellt, bestand die Aufgabe, ausschließlich kristalline Salze des Acetoacetamid-N-sulfofluorids herzustellen.

Diese Aufgabe konnte erfindungsgemäß hauptsächlich durch den Ersatz der organischen N-Basen in dem Verfahren gemäß DE-OS 2 453 063 durch anorganische Basen gelöst werden.

Erfindungsgegenstand ist daher ein Verfahren zur Herstellung kristalliner Salze des Acetoacetamid-N-sulfofluorids durch Umsetzung von Amidosulfofluorid mit Diketen in Gegenwart von Basen in einem inerten organischen Lösungsmittel ; das Verfahren ist dadurch gekennzeichnet, daß man als Basen anorganische Basen verwendet.

Bevorzugte anorganische Basen sind die Carbonate und Hydrogencarbonate der Alkalimetalle (Lithium, Natrium, Kalium, Rubidium und Cesium), wobei Kaliumcarbonat und Kaliumhydrogencarbonat — vor allem Kaliumcarbonat — besonders bevorzugt sind. Die Menge an einzusetzender anorganischer Base beträgt vorteilhaft mindestens etwa 1 Grammäquivalent pro Mol Amidosulfofluorid. Vorzugsweise arbeitet man jedoch mit einem Basenüberschuß bis zu etwa 20 %, insbesondere nur bis zu etwa 10 %. Im allgemeinen ist es zweckmäßig und ausreichend, die Menge an anorganischer Base so zu bemessen, daß das erfindungsgemäße Reaktionsgemisch nach beendeter Umsetzung einen pH-Wert von mindestens = 7 aufweist, gemessen mit feuchtem pH-Indikatorpapier.

Nach dem Verfahren werden ausschließlich feste kristalline Salze des Acetoacetamid-N-sulfofluorids in Ausbeuten durchweg zwischen etwa 85 und 95 % d. Th., bezogen auf Amidosulfofluorid, erhalten. Ein besonderer Vorteil gegenüber dem Verfahren der vorerwähnten DE-OS 2 453 063 besteht auch in dem Einsatz der (gegenüber den organischen N-Basen) einfacheren und billigeren anorganischen Basen. Wenn schließlich — ausgehend von Amidosulfofluorid — etwa der Süßstoff Acesulfam hergestellt wird, werden über das erfindungsgemäße Verfahren pro Mol Amidosulfofluorid an Basen insgesamt etwa 2 (oder nur wenig mehr als 2) Grammäquivalente einer Kaliumbase benötigt, während über das Verfahren der DE-OS 2 453 063 mindestens ein Grammäquivalent einer organischen Stickstoffbase und mindestens etwa 2 Äquivalente einer Kaliumbase erforderlich sind, wobei die organische Stickstoffbase in einem zusätzlichen Verfahrensschritt wieder zurückgewonnen werden muß.

Der Erhalt der festen kristallinen Salze des Acetamido-N-sulfofluorids in glatter Reaktion mit hohen Ausbeuten ist sehr überraschend, weil normalerweise die Salze organischer N-Basen (Ammoniumsalze) und anorganische Salze — insbesondere Alkalisalze — mit der jeweils gleichen anionischen Salzkomponente von gleicher physikalischer Konsistenz sind. Außerdem war der glatte und schnelle Reaktionsverlauf auch deswegen unerwartet, weil sich die erfindungsgemäß verwendeten anorganischen Basen in dem organischen Reaktionsmedium kaum lösen (heretogene Reaktion !).

Die erfindungsgemäße Reaktion verläuft — analog der Reaktion gemäß DE-OS 2 453 063 — nach folgendem Reaktionsschema (mit Kaliumcarbonat als anorganischer Base) :

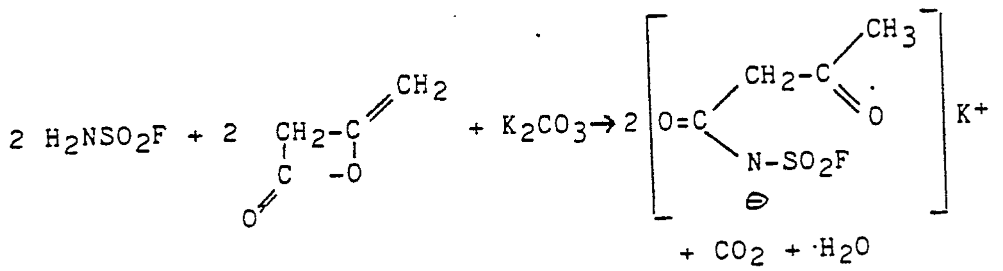

Die Menge des eingesetzten Diketens soll (im Verhältnis zu dem Reaktionspartner Amidosulfofluorid) mindestens etwa äquimolar sein. Bevorzugt ist der Einsatz eines bis zu etwa 30 Mol-%igen Überschusses, insbesondere eines Überschusses zwischen etwa 10 und 20 Mol-%. Höhere als etwa 30 Mol-%ige Überschüsse sind möglich, bringen aber keinen Vorteil.

Als inerte organische Lösungsmittel kommen praktisch alle organischen Lösungsmittel in Betracht, welche mit den Ausgangs- und Endstoffen (einschließlich den anorganischen Basen) nicht in unerwünschter Weise reagieren. Folgende organische Lösungsmittel sind als hier beispielhaft in Frage kommend zu nennen :

Niedere aliphatische Ketone, vorzugsweise mit 3 bis 6 C-Atomen, wie z. B. Aceton, Methylethylketon ;

aliphatische Ether, vorzugsweise cyclische aliphatische Ether mit 4 bis 5 C-Atomen wie z. B. Tetrahydrofuran, Dioxan ;

N-alkylsubstituierte Amide niederer aliphatischer Carbonsäuren, vorzugsweise solche mit bis zu insgesamt 7 C-Atomen wie z. B. Dimethylformamid ;

aliphatische Sulfoxide, vorzugsweise Dimethylsulfoxid und aliphatische Sulfone, vorzugsweise Sulfolan.

Besonders bevorzugte Lösungsmittel aus der vorstehenden Aufzählung sind aus Gründen der Reaktionsführung solche, deren Siedepunkte im Bereich der gewünschten Reaktionstemperatur liegen ; dies sind hier hauptsächlich Aceton, Acetonitril und Dimethylformamid, vor allem Aceton.

Die Lösungsmittel können sowohl einzeln als auch in Mischung eingesetzt werden.

Das Mengenverhältnis von Reaktions-Ausgangsstoffen zu Lösungsmittel kann in weiten Grenzen variieren ; im allgemeinen liegt das Gewichtsverhältnis bei etwa 1 : (2-20).

Die Reaktionstemperatur liegt praktisch im gleichen Bereich wie auch diejenige des Verfahrens gemäß DE-OS 2 453 063 ; d. s. also Temperaturen im allgemeinen zwischen etwa — 30 und + 100 °C, vorzugsweise zwischen etwa — 20 und + 50 °C, insbesondere zwischen etwa — 10 und + 30 °C.

Das Verfahren wird normalerweise bei Atmosphärendruck durchgeführt.

Die Reaktionszeit kann innerhalb weiter Grenzen schwanken ; sie liegt im allgemeinen zwischen etwa 0,5 und 12 Stunden.

Das erfindungsgemäße Verfahren wird beispielsweise so durchgeführt, daß man die anorganische Base in dem inerten organischen Lösungsmittel bei Raumtemperatur oder darunter vorlegt. Unter guter Durchmischung gibt man zunächst das Amidosulfofluorid und dann das Diketen in der Weise zu, daß die Reaktionstemperatur auf dem gewünschten Wert gehalten werden kann. Man rührt das Reaktionsgemisch anschließend, gegebenenfalls unter Erhöhung der Temperatur, weiter, bis die charakteristischen IR-Banden des Diketens bei 5.2 und 5.3 μ im Umsetzungprodukt nicht mehr nachweisbar sind. Die entstandenen Salze des Acetoacetamid-N-sulfofluorids mit der eingesetzten anorganischen Base fallen als farblose kristalline Produkte an.

Zur Charakterisierung der Reaktionsprodukte dienen z. B.

a) die IR-Spektren, die mit Spektren authentischen Materials (hergestellt aus Acetoacetamid-N-sulfofluorid und der entsprechenden anorganischen Base) verglichen werden, und

b) die Überführung der Produkte in den Süßstoff Acesulfam mittels 1 Mol methanolischer KOH.

Die letztere Reaktion wird bevorzugt zur Ausbeutebestimmung herangezogen.

Gewünschtenfalls ist natürlich aus den verfahrensgemäß erhaltenen Salzen auch das freie Acetoacetamid-N-sulfofluorid auf übliche Weise herstellbar, z. B. durch Ansäuern der Salze mit kalter Salzsäure und Isolierung des erhaltenen Acetoacetamid-N-sulfofluorids durch Extraktion, z. B. mittels Essigsäureethylester.

Die nachfolgenden Beispiele sollen die Erfindung nun näher erläutern.

### Beispiel 1

76 g (0,55 Mol) Kaliumcarbonat-Pulver wurden in 500 ml Aceton bei 0 °C vorgelegt. Nach der Zugabe von 57,8 ml (1 Mol) Amidosulfofluorid wurden 84,3 ml (1,1 Mol) Diketen innerhalb 15 Minuten zugetropft. Anschließend wurde 30 Minuten bei 0 °C nachgerührt ; dann wurde die Temperatur auf maximal 30 °C ansteigen gelassen. Nach ca. 30 Minuten war die exotherme Reaktion und nach weiteren 60 Minuten die $CO_2$-Entwicklung beendet (IR-spektroskopisches Fehlen der charakteristischen Banden des Diketens bei 5.2 und 5.3 μ). Das entstandene Kaliumsalz des Acetoacetamid-N-sulfofluorids field als farbloses kristallines Pulver an, wurde abgesaugt und mit wenig Aceton gewaschen. Es war in allen Eigenschaften identisch mit authentischem Material, das aus Acetoacetamid-N-sulfofluorid und Kaliumcarbonat hergestellt wurde.

Zur Ausbeutebestimmung wurde das erhaltene Kaliumsalz des Acetoacetamid-N-sulfofluorids mit 1 Äqu. 4- bis 6-molarer methanolischer KOH-Lösung verrührt und so in den Süßstoff 6-Methyl-3,4-dihydro-1,2,3-oxathiazin-4-on-2,2-dioxid-Kaliumsalz überführt.

Die Ausbeute betrug 93 % (d. Th.).

### Beispiel 2

110,1 g (1.1 Mol) Kaliumhydrogencarbonat-Pulver wurden in 500 ml Acetonitril bei 0 °C vorgelegt. Nach der Zugabe von 57.8 ml (1 Mol) Amidosulfofluorid wurden 84.3 ml (1,1 Mol) Diketen innerhalb 15 Minuten zugetropft. Die weitere Reaktionsführung, Aufarbeitung und Ausbeutebestimmung erfolgte analog Beispiel 1.

Ausbeute 89 % (d. Th.)

### Beispiel 3

76 g (0,55 Mol) Kaliumcarbonat-Pulver wurden in 300 ml Dimethylformamid bei 0 °C vorgelegt. Nach der Zugabe von 57,8 ml (1 Mol) Amidosulfofluorid wurden 84,3 ml (1,1 Mol) Diketen innerhalb 15 Minuten zugetropft. Anschließend wurde 30 Minuten bei 0 °C und dann 90 Minuten bei maximal 30 °C nachgerührt. Nach der Zugabe von 500 ml Aceton wurde das Kaliumsalz des Acetoacetamid-N-sulfofluorids abgesaugt und mit wenig Aceton nachgewaschen. Die Charakterisierung und Ausbeutebestimmung erfolgte wie in Beispiel 1.

Ausbeute 90 % (d. Th.).

### Beispiel 4

58,3 g (0,55 Mol) Natriumcarbonat-Pulver wurden in 500 ml Aceton bei 0 °C vorgelegt. Nach der Zugabe von 57,8 ml (1 Mol) Amidosulfofluorid wurden 84,3 ml (1,1 Mol) Diketen innerhalb 15 Minuten zugetropft. Anschließend wurde 30 Minuten bei 0 °C und dann 90 Minuten bei maximal 30 °C nachgerührt. Nach der Zugabe von 500 ml Diethylether wurde das Natriumsalz des Acetoacetamid-N-sulfofluorids abgesaugt. Die Umsetzung mit 1 Äqu. 4- bis 6-molarer methanolischer KOH-Lösung lieferte das Kaliumsalz des Süßstoffs, das geringe Mengen Natriumsalz des Süßstoff enthielt. Daher wurde zur Ausbeutebestimmung das Salzgemisch in wenig Wasser gelöst, mit konzentrierter Salzsäure auf pH 1 gestellt und die Süßstoffsäure 6-Methyl-3,4-dihydro-1,2,3-oxathiazin-4-on-2,2-dioxid mit Ethylacetat

extrahiert.
Ausbeute 84 %.

Beispiel 5

40,6 g (0,55 Mol) Lithiumcarbonat-Pulver wurden in 500 ml Aceton bei 0 °C vorgelegt. Nach der Zugabe von 57,8 ml (1 Mol) Amidosulfofluorid wurden 84,3 ml (1,1 Mol) Diketen innerhalb 15 Minuten zugetropft. Die weitere Reaktionsführung, Aufarbeitung und Ausbeutebestimmung erfolgte analog Beispiel 4.
Ausbeute 86 % (d. Th.).

**Patentansprüche**

1. Verfahren zur Herstellung kristalliner Salze des Acetoacetamid-N-sulfofluorids durch Umsetzung von Amidosulfofluorid $H_2NSO_2F$ mit Diketen, in Gegenwart von Basen in einem inerten organischen Lösungsmittel, dadurch gekennzeichnet, daß man als Basen anorganische Basen verwendet.

2. Verfahren nach Anspruch 1, dadurch gekennzeichnet, daß man als anorganische Basen Alkalicarbonate und/oder -hydrogencarbonate, insbesondere Kaliumcarbonat, verwendet.

3. Verfahren nach den Ansprüchen 1 bis 2, dadurch gekennzeichnet, daß man die anorganischen Basen in einer Menge von mindestens 1 Grammäquivalent, vorzugsweise einem Überschuß bis zu 20 %, insbesondere nur bis zu 10 %, bezogen auf ein Mol Amidosulfofluorid, verwendet.

4. Verfahren nach den Ansprüchen 1 bis 3, dadurch gekennzeichnet, daß man das Diketen in mindestens äquimolarer Menge, vorzugsweise in einem bis zu 30 Mol-%igen Überschuß, insbesondere einem Überschuß zwischen 10 und 20 Mol-%, bezogen auf das Amidosulfofluorid, verwendet.

5. Verfahren nach den Ansprüchen 1 bis 4, dadurch gekennzeichnet, daß man als inerte organische Lösungsmittel aliphatische Ketone, Ether, Nitrile, Carbonsäureamide, Sulfoxide und/oder Sulfone verwendet.

6. Verfahren nach den Ansprüchen 1 bis 5, dadurch gekennzeichnet, daß man als inerte organische Lösungsmittel Aceton, Acetonitril und/oder Dimethylformamid, insbesondere Aceton, verwendet.

7. Verfahren nach den Ansprüchen 1 bis 6, dadurch gekennzeichnet, daß man die Umsetzung bei Temperaturen zwischen —30 und + 100 °C, vorzugsweise zwischen —20 und + 50 °C, insbesondere zwischen —10 und + 30 °C, durchführt.

**Claims**

1. A process for preparing crystalline salts of acetoacetamide-N-sulfofluoride by reacting amidosulfofluoride $H_2NSO_2F$ with diketene in the presence of bases in an inert organic solvent, characterized in using as bases inorganic bases.

2. The process as claimed in claim 1, characterized in using as inorganic bases alkali metal carbonates and/or hydrogen carbonates, in particular potassium carbonate.

3. The process as claimed in claims 1 to 2, characterized in using the inorganic bases in an amount of at least 1 gram equivalent, preferably an excess up to 20 %, in particular only up to 10 %, based on one mol of amidosulfofluoride.

4. The process as claimed in claims 1 to 3, characterized in using the diketene in an at least equimolar amount, preferably in an excess of up to 30 mol %, in particular an excess between 10 and 20 mol %, based on the amidosulfofluoride.

5. The process as claimed in claims 1 to 4, characterized in using as inert organic solvents aliphatic ketones, ethers, nitriles, carboxamides, sulfoxides and/or sulfones.

6. The process as claimed in claims 1 to 5, characterized in using as inert organic solvents acetone, acetonitrile and/or dimethylformamide, in particular acetone.

7. The process as claimed in claims 1 to 6, characterized in carrying out the reaction at temperatures between —30 and + 100 °C, preferably between —20 and + 50 °C, in particular between —10 and + 30 °C.

**Revendications**

1. Procédé de préparation de sels cristallisés du N-fluorosulfonyl -acétoacétamide par réaction de la fluorosulfonylamine $H_2NSO_2F$ avec le dicétène en présence de bases dans un solvant organique inerte, procédé caractérisé en ce que l'on utilise comme bases des bases minérales.

2. Procédé selon la revendication 1 caractérisé en ce que l'on utilise comme bases minérales des carbonates et/ou des hydrogénocarbonates de métaux alcalins, notamment le carbonate de potassium.

3. Procédé selon les revendications 1 et 2 caractérisé en ce que l'on ajoute les bases minérales dans

une proportion d'au moins 1 équivalent-gramme et de préférence en un excès pouvant s'élever jusqu'à 20 %, en particulier jusqu'à 10 % seulement, par mole de l'amidosulfofluorure.

4. Procédé selon les revendications 1 à 3, caractérisé en ce que l'on ajoute le dicétène dans la proportion au moins équimolaire et de préférence en un excès pouvant s'élever jusqu'à 30 moles %, notamment en un excès de 10 à 20 moles %, par rapport à l'amidosulfofluorure.

5. Procédé selon les revendications 1 à 4, caractérisé en ce que l'on utilise comme solvants organiques inertes des cétones aliphatiques, des éthers, nitriles, amides d'acides carboxyliques, sulfoxydes et/ou sulfones.

6. Procédé selon les revendications 1 à 5, caractérisé en ce que l'on utilise comme solvant organique inerte l'acétone, l'acétonitrile et/ou le diméthylformamide, en particulier l'acétone.

7. Procédé selon les revendications 1 à 6, caractérisé en ce que l'on effectue la réaction à des températures de —30 à + 100 °C, de préférence de —20 à + 50 °C, et plus spécialement de —10 à + 30 °C.